# EUROPEAN PATENT APPLICATION

(11) **EP 1 772 522 A1**
(43) Date of publication of application: **11.04.2007**
(21) Application number: 05077246.6
(22) Date of filing: 04.10.2005
(51) Int. Cl.: C12Q 1/68, C12Q 1/22

(54) **Control of preservation by biomarkers**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: Keijser, Bart Jan Frederik, 1216 AG Hilversum (NL)
(74) Representative: Winckels, Johannes Hubertus F.

(57) **Abstract**

The invention lies in the field of microbiology, more particularly in the field of (food) preservation and testing of viability of microbiological spores. It is shown that measurement of the integrity of both rRNA and mRNA in spores is an accurate indicator of viability. Nucleotide assays then form a significant improvement over the state of the art assays for viability.

## Description

The invention lies in the field of microbiology, more particularly in the field of (food) preservation and testing of viability of microbiological spores.

Since the development of pasteurisation no real break-through has found place in the field of food preservation. The most common preservation process is heat treatment and the effectiveness of preservation is nowadays determined by a trial and error method. Thus, present preservation methods are directed to a 'worst case' scenario, in which the most infected sample is taken as standard for subsequent preservation treatments. One of the reasons for this is that a detailed insight in the process of preservation lacks: current methodologies are not capable of a reliable and quick assessment of the micro-organisms and their viability in relation to the preservation process. Hitherto, the inactivation of contaminating micro-organisms is measured indirectly: by determining the growth of surviving micro-organisms after heat treatment.
This method has two large disadvantages:
- it is very time consuming (often results are only available after more than two days);
- if no survivors are measured, it cannot be determined whether the applied treatment was just barely sufficient or meant an overkill.

The worst case scenario in practice means that often more energy is used for the heat treatment than would have been necessary, because either the contamination was less than expected or the micro-organisms in the sample were more heat-sensitive than those on which the treatment was based.

Thus, there is a large need for rapid microbiological testing of (heat) treated samples to assess the quality and effect of the preservation treatment.

In the last couple of years genomic techniques have enabled a relatively rapid and specific approach to microbiological assaying. With these techniques it is possible to detect even small amounts of (micro)organisms within a sample, and it has become possible to discriminate between micro-organisms on a species or subspecies level. However, especially in heat (or other stress) treated food, the most important safety challenge is not formed by surviving micro-organisms, but by the spores that most of the spore-forming micro-organisms form under unfavourable (stress) conditions. Spore-forming micro-organisms, such as *Bacillus,* determine for a major part the shelf-life of heat-preservation treated food. One spore is formed from one vegetative bacterium. Each spore is composed of a protoplast, a protoplast membrane, cortex and three coat layers (Turnbill, 1996). This multilayered outer shell excludes macromolecules. An additional heat and chemical resistant component in the cortex, peptidoglycan, increases the spore's resiliency (Turnbill, 1996). Bacillus spores central protoplast contains dipicolinic acid, (DPA), the component necessary for high temperature tolerance (Turnbill, 1996). Slieman et al. (2001) found that approximately 10 percent of a spore's dry weight is DPA. DPA exists as a calcium complex. Rosen et al. (1997) developed a terbium chloride assay to identify and quantify endospore concentrations utilizing this DPA-Ca++ complex. The reaction between the calcium dipicolinic acid complex and terbium chloride results in a terbium (III) anion. This terbium anion is photoluminescent when in the presence of the DPA-Ca++ complex and is easily recognized.

Several species of *Bacillus* are important to human and animal health. *B. anthracis,* the agent of anthrax, is a zoonotic disease which primarily affects grazing animals and can also be a dangerous pathogen to humans. *B*. *anthracis* spores are known to survive along livestock trails in the United States causing frequent outbreaks in states from Texas to South Dakota (CDC, 2001). Members of the *B. cereus* group can cause food poisoning in humans and related forms (eg. *B. thuringiensis)* are used in biological control of insect pests.

As mentioned above, the protoplast is enveloped by the cortex, followed by three protein coats (Turnbill, 1996). However, members of the *B. cereus* group, which includes, *B. anthracis,* have an additional protective layer, the outer-most exosporium. These multilayered outer structures, which can make up half of the spore's weight, provide protection for the spores from chemical, physical and enzymatic degradation (Turnbill, 1996).

There are several reliable assays used to characterize and identify spores. Genetic identification relies on PCR- DNA sequencing to identify the species by their nucleotide sequences (Hansen,B., Leser,T., Hendriksen, N. 2001. Polymerase chain reaction assay for the detection of Bacillus cereus group cells. FEMS Microbiology Letters 202: 209-213.; Kolbert, C., Persing, D. 1999. Ribosomal DNA sequencing as a tool for identification of bacterial pathogens. Microbiology 2:299-305.). Phenotypic identification relies on physiological profiling such as the Sherlock Microbial Identification System (MIDI Inc., Newark, DE), and BIOLOG, (Biolog, Inc., Hayward, CA). The MIDI system utilizes fatty acid analysis of the bacterium, and assigns a numerical value to the results, called a similarity index. This similarity index is then compared to an internal library which chooses the most similar identities for the microbes in question. BIOLOG is a cell-based test for utilization of carbon sources. It requires a 96-well plate with multiple carbon sources from which color changes are compared to a Biolog library to determine the identification of the bacterium. Steady-state fluorescence is a powerful tool for distinguishing differences in molecules and macromolecules and the technique may prove useful in examining spores. Fluorescence spectroscopy utilizes emission peaks to characterize spores. It is a sensitive technique because excitation is performed at a single wavelength. The resulting emission data are recorded at a longer wavelength. Bronk and Reinisch (1993) concluded that initial microbial identification could be generated using fluorescence spectroscopy. All spore assays are influenced by environmental contaminants (Kuske, et al., 1998, Balser, et al. 2002, Gamo et al. 1999) and each would benefit from isolation, concentration and purification.

One technique that may aid in spore isolation and concentration from environmental samples is to use partitioning into hexadecane or some similar hydrophobic material whereby hydrophobic spores would partition into the hydrocarbon and hydrophilic spores would remain in the aqueous phase.

Thus spores are commonly more heat-resistant than the cells of the micro-organism, also because they do not require an active metabolism during the spore stage. Yet, on return of favourable conditions the spores can develop into colonizing micro-organisms and then are able to cause enormous healthrisks. Until now no genomics-based techniques have been developed to assay the amount and viability of spores after preservation treatment.

### SUMMARY OF THE INVENTION

The invention now provides for an easy and reliable assay to measure the viability and/or detoriation (e..g. by damaging) of spores during or after preservation treatment. The assay uses biomarkers, comprising RNAs, more especially ribosomal RNAs (rRNAs) and messenger RNAs (mRNAs). It has now been found that preservation treatments such as a heat treatment or pressure degrades these RNAs and also that the sensitivity towards degradation varies within these ribonucleotides Further, the invention provides for a system to type the spores, which are present as food contaminants.

Thus, the invention provides a method to measure viability of heat, pressure or radiation treated bacterial spores comprising measuring the degree of degradation of RNA by said heat or radiation treatment in said spores, particularly wherein the RNA is either ribosomal RNA (rRNA), messenger RNA (mRNA) or both, more particularly wherein the treatment is heat treatment.

Another embodiment of the invention is a method to assay the effect of heat preservation methods by performing a method as described above.

Alternatively, the invention provides a method to assay bacterial contamination after heat or radiation preservation by performing a method according to the invention.

The invention also provides a heat preservation method comprising
a) maintaining a sample at a certain temperature for a certain time;
b) performing a method according to the invention.

Another embodiment of the present invention is formed by the use of RNA in microbial spores as a biomarker for viability.

Further, the invention provides a method for typing microbial spores comprising the steps of:
a) isolating the RNA form said spores;
b) optionally translate the RNA to DNA by reverse transcriptase and/or amplify the RNA or DNA; and
c) hybridising said RNA with type specific nucleotide probes or performing a sequence analysis of said RNA;
   preferably wherein the RNA is mRNA.

### LEGENDS TO THE FIGURES

Fig. 1 shows survival of *B. subtilis* 168 spores after heat treatment at the indicated temperatures during the time period depicted on the X-axis. The Y-axis indicates logarithmically the number of colony forming units (cfu).

Fig. 2 shows a Bioanalyzer pseudogel of RNA isolated from *B*. *subtilis* 168 spores treated at three different temperatures (90 °C, 98 °C and 105 °C) each during four different time periods (2, 5, 10 and 20 minutes), indicating the integrity of the RNA. The left lane represents molecular weight control.

Fig. 3 shows the results of survival counts of two *B. subtilis* strains (168 and A163) which have been heat treated at the indicated temperatures (X-axis) during a period of 5 minutes. Y-axis as in Fig. 1.

Fig. 4 shows a Bioanalyzer pseudogel of RNA isolated from the spores. Treatments identical as in Fig. 3.

Fig. 5 Shows a clusteranalysis of spore mRNAs (in duplo, indicated on the right) measured during germination. Red color indicates presence of transcript, green color indicates absence.

Fig. 6 shows ethidium bromide stained gels of three PCR products (Bs-2 (=*coxA*), Bs-4 (*=ykzE*) and Bs-7 (*=sspE*) obtained through RT-PCR from the spore mRNA. In each panel the left lane shows the molecular weight marker.

Fig. 7 indicates survival *of B. subtilis* 168 spores after heat treatment at 98 °C for the indicated time periods (X-axis). Y-axis as in Fig. 1.

Fig. 8 depicts the results of the quantitative analysis of degradation of spore rRNAs and mRNAs. The bars indicate the ¹⁰log value of the decrease in RNA concentration measured in treated spores in comparison with untreated spores. Spores and treatment s in Fig. 7.

### DETAILED DESCRIPTION OF THE INVENTION

The presence of messenger RNA is microbial spores has been disputed. Some old literature indicates that spores contain mRNAs (Aronson A.J., 1965, Mol. Biol. 13:92-104; Jeng Y.H. and Doi R.H., 1974, J. Bacteriol. 119:514-521), while other authors report an absence (Halvorson HO, Vary JC, Steinberg W., J, (1966). Developmental changes during the formation and breaking of the dormant state in bacteria. Annu Rev Microbiol 20:169-88; R. H. Doi and R. T. Igarashi (1964) RIBONUCLEIC ACIDS OF BACILLUS SUBTILIS SPORES AND SPORULATING CELLS. Bacteriol. February; 87(2): 323-328.). However, nowhere in the art identification of spores on basis of mRNA or other RNA sequences is taught.

It has now appeared that spores contain ribosomal RNAs (more particularly the 16S and 23S rRNAs) and also mRNAs for about 20 gene products. The latter finding is surprising, since it was generally thought that only de *novo* transcription takes place in spores (during germination). This small group of mRNAs is generated during the last phase of sporulation and is in general rapidly degraded during germination. The function of these transcripts and why they are maintained in the spore is as of yet unclear. A list of the mRNAs that are found in spores is given in Table 1.

**Table 1: List of spore mRNA found in spores of B. subtilis 168**

| Gene Details | Gene name |
|---|---|
| BG14189 | *yozQ* |
| BG14179 | *sspN* |
| BG14174 | *sspJ* |
| BG13937 | *ytzC* |
| BG13861 | *ythQ* |
| BG13859 | *ythC* |
| BG13782 | *coxA* |
| BG13430 | *ymfJ* |
| BG13334 | *ykzF* |
| BG13333 | *ykzE* |
| BG13008 | *yhdB* |
| BG12879 | *yfhD* |
| BG11921 | *sspP* |
| BG11920 | *sspO* |
| BG11806 | *tlp* |
| BG11670 | *yqfX* |
| BG11600 | *yhcV* |
| BG11595 | *yhcQ* |
| BG11592 | *yhcN* |
| BG10789 | *sspE* |
| BG10108 | *sspF* |

The presence of both rRNA and mRNA enables using molecular biological techniques to study survival and/or heat resistance in spores during radiation and heat treatment (e.g. for preservation). One of the findings of this invention is that both rRNA and mRNA are being degraded during heat treatment, which degradation coincides with the loss of viability of the spores. Degradation predominantly depends on the type of micro-organism: in spores of a more heat resistant micro-organism the degradation of the RNA starts at higher temperatures. Thus, the degradation of RNA is an excellent parameter for determination of the viability and/or (sublethal) damage of the spores.

Molecular biological assays for the detection of RNA and/or determination of the length of the RNA fragments can now be used to assess the viability of spores in (food and other) samples. This is not only useful during development of preservation methods on basis of heat treatment, but these assays can also be applied in the regular testing of the condition of food samples, e.g. by the commodity inspection department.

Secondly, the presence of mRNA in spores can be used to generate assays for the identification and classification of spores in samples. It is envisaged that typical differences in nucleotide sequence or polymorphisms between species of bacteria can be used to type the spores. This would make redundant the time consuming culturing of the spores and typing of the resulting colonies.

Typically, for the molecular biological methods of the invention, first the RNA of the spores has to be isolated from the sample. To enrich the sample for spores, methods which are well known to a person skilled in the art can be used (e.g. Vaerewijck, M.J.M. et al. (2001) J. Appl. Microbiol. 91:1074-1084). Then, spores are lysed and RNA is extracted using commonly known techniques. RNA isolation from spores can, for instance, be done through a commercially available kit, such as the BI0101-FASTRNA Pro Blue kit (QBIOGENE cat no 6025-050). To remove superfluous DNA from the RNA, the extracted sample can be treated further with a DNAse1, such as present in the TURBO DNA-free kit (AMBION, cat no 1907).

Determination of the length of the RNAs can be performed on normal gels, such as agarose gels, but it is also possible to analyse the integrity of the RNA on a bioanalyzer RNA chip, such as a RNA 6000 Nano LabChip (Agilent Technologies cat no 5065-4476) using a 2100 bioanalyzer (Agilent Technologies, cat no G2940CA). The integrity of the isolated RNA samples is normally derived from the relative intensity of the bands for the ribosomal subunits (16S and 23S). When the RNA is intact the ratio between 23S and 16S is about 2:1. On RNA degradation this ratio shifts usually in favour of 16S RNA and increasingly RNA debris is found.

In order to make detection easier the nucleotides in the sample can be amplified, e.g. through an RNA amplification kit, such as Nucleic Acid Sequence Based Amplification (NASBA),. Alternatively, it is also possible to convert the RNA into DNA by using a reverse transcriptase and then to amplify the DNA by PCR techniques. Methods of the invention can in principle be performed by using any nucleic acid amplification method, such as the Polymerase Chain Reaction (PCR; Mullis 1987, U.S. Pat. No. 4,683,195, 4,683,202, en 4,800,159) or by using amplification reactions such as Ligase Chain Reaction (LCR; Barany 1991, Proc. Natl. Acad. Sci. USA 88:189-193; EP Appl. No., 320,308), Self-Sustained Sequence Replication (3SR; Guatelli et al., 1990, Proc. Natl. Acad. Sci. USA 87:1874-1878), Strand Displacement Amplification (SDA; U.S. Pat. Nos. 5,270,184, en 5,455,166), Transcriptional Amplification System (TAS; Kwoh et al., Proc. Natl. Acad. Sci. USA 86:1173-1177), Q-Beta Replicase (Lizardi et al., 1988, Bio/Technology 6:1197), Rolling Circle Amplification (RCA; U.S. Pat. No. 5,871,921), Cleavage Fragment Length Polymorphism (U.S. Pat. No. 5,719,028), Isothermal and Chimeric Primer-initiated Amplification of Nucleic Acid (ICAN), Ramification-extension Amplification Method (RAM; U.S. Pat. Nos. 5,719,028 and 5,942,391), LATE-PCR (Sanchez, J.A. et al (2004) PNAS USA 101:1933-1938) or other suitable methods for amplification of DNA.

For quantitative assays a Real Time-PCR or a semi-RT-PCR can be used. All of these amplification techniques are known by the person skilled in the art, and application of those can be found in the Examples, hereunder.

The detection of the amplification products can in principle be accomplished by any suitable method known in the art. The detection fragments may be directly stained or labelled with radioactive labels, antibodies, luminescent dyes, fluorescent dyes, or enzyme reagents. Direct DNA stains include for example intercalating dyes such as acridine orange, ethidium bromide, ethidium monoazide or Hoechst dyes. Alternatively, the DNA fragments may be detected by incorporation of labelled dNTP bases into the synthesized DNA fragments. Detection labels which may be associated with nucleotide bases include e.g. fluorescein, cyanine dye or BrdUrd.

Amplification can be used to detect the integrity of the RNA sequences because amplification primers can be chosen in such a way that only (nearly) complete, i.e. not yet degraded sequences will be amplified. A suitable way to accomplish this is by choosing a forward amplification primer which corresponds with a sequence at the 5' end of the sequence which needs to be amplified. Consequently, the backward or reverse primer should be chosen to correspond with a sequence at the 3' end of the sequence to be amplified. Only if forward and reverse primer anneal to one and the same (intact) RNA stretch amplification will occur; if the target sequence has been degraded by heat or radiation treatment a fragment will at the utmost only contain the recognition site for one of the set of primers and no amplification will take place.

The term "primer" as used herein refers to an oligonucleotide which is capable of annealing to the amplification target allowing a DNA polymerase to attach thereby serving as a point of initiation of DNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primers will depend on many factors, including temperature and source of primer. A "pair of bi-directional primers" as used herein refers to one forward and one reverse primer as commonly used in the art of DNA amplification such as in PCR amplification.

If the nucleotide sequences from the sample are subjected to amplification for the detection of RNA integrity, it is a prerequisite that from each micro-organism (i.e. from each spore) at least one sequence is amplified. Therefore, primer sets should be chosen which recognise all possible present micro-organisms. This can be achieved by identifying conserved sequences, which are shared by all the micro-organisms. Since it is sometimes impossible to cover all micro-organisms with one and the same primer set, it is envisaged that more than one pair of bidirectional primers is needed to be present in de amplification reaction mixture to enable amplification of at least one sequence from all possibly present micro-organisms. Luckily, the 16S and 23S rRNA are rich in stretches of conserved sequences. Alternatively, or in addition to that, it is also possible to use degenerated primers, i.e. primers which do not or less suffer from any mismatches between the primer sequence and the target sequence on the gene to be amplified, thereby allowing for hybridisation of the primer to a target sequence with a lower homology.

Another method to determine the viability and/or amount of damage to the spores is to assess for the ratio of intact DNA versus intact RNA. As already mentioned in the introduction, the spores also contain DNA, which has other temperature sensitivity than RNA. In a control experiment it can be determined for a particular bacterial strain how the ratio of DNA:RNA develops when the spores are subjected to heat treatments, and at which ratio the spores are lethally damaged or lost their viability. A simple detection of the amounts of DNA and the amounts of RNA can then be used to determine viability and/or damage.

Another embodiment of the present invention involves the detection of the reaction products obtained by the above described DNA amplification reaction. Amplification of the nucleotides sequences in the sample is especially indicated when the detection of nucleotides is used for typing the identity of the spores, i.e. the micro-organism which has generated the spores. Suitable detection procedures for use in the typing aspect of present invention may for example comprise immobilization of the amplicons (the amplified sequences) and probing the DNA sequences thereof by e.g. Southern blotting. Other formats may comprise an EIA format. To facilitate the detection of binding, the type-specific probes may comprise a label moiety such as a fluorophore, a chromophore, an enzyme or a radio-label, so as to facilitate monitoring of binding of the probes to the reaction product of the amplification reaction. Such labels are well-known to those skilled in the art and include, for example, fluorescein isothiocyanate (FITC), β-galactosidase, horseradish peroxidase, streptavidin, biotin, digoxigenin, ³⁵S or ¹²⁵I. Other examples will be apparent to those skilled in the art.

Although other methods may be employed, a method of typing according to the invention is preferably performed by hybridisation of PCR products to type-specific probes immobilized to beads or RLB filters, such as recently described for e.g. a consensus PCR method (van den Brule et al., 2002, J. Clin. Microbiol. 40, 779-787). For this purpose RLB probes are preferably synthesized with a 5' amino group for subsequent immobilization on e.g. carboxyl-coated nylon membranes. The advantage of such a format is the ease of the system and its speed, thus allowing for high throughput sample processing.

The various types of micro-organisms that occur in a sample to be tested may be detected by using a plurality of type-specific detection probes that, individually, bind only to the amplicon of one specific micro-organism. Thus, by amplifying the DNA sequence region between the two primer target regions as defined herein, followed by probing for the unique sequences of each individual micro-organism, a method is provided for typing of the various micro-organisms. Typing can be done on the rRNA, but is preferably performed on the mRNA, since a lot more variation between the micro-organisms is present in said mRNA, which increases the possibility for generating specific probes.

Alternatively, typing can also be achieved by amplification alone: then, instead of using a universal pair of bidirectional primers (or sets of bidirectional primers), specific primer sets can be used. If amplification products are formed on basis of such a specific amplification reaction a positive detection (and typing) is achieved. Again, on basis of the larger variability within the mRNAs, specific primer pairs are preferably designed on basis of unique mRNA sequences. Advantageously, multiplex amplification, i.e. an amplification procedure which comprises multiple specific pairs of bidirectional primers directed to different mRNAs of different micro-organisms, is used. However, in such a system, care should be taken that each of the amplification reaction products is differently labelled to enable specific detection. Systems and labels for multiplex amplification and detection are known to a person skilled in the art, e.g. Hardenbol, P. et al. (2003) Nat. Biotechnol. 21:673-678; Baner, J., et al. (2003) Nucleic Acids Res. 31:e103; Alsmadi, O.A., et al. (2003) BMC Genomics 4:e21; Denef, et al., (2003) Environ. Microbiol. 5:933-943).

Use of the methods of the invention can of course be found in the food-industry. Not only detection of contamination of actual foodstuffs can be performed through the methods of the invention, these methods also are a useful tool in the development of (new) preservation methods for different types of samples (predominantly food). The methods enable monitoring of preservation processes and quick feedback on the results thereof. Also, as a result of better preservation techniques, next to a decrease of product loss also savings of energy and water can be obtained.

Besides the food industry, the present invention can also be applied in other areas where heat and/or radiation and/or pressure are used for sterilisation (i.e. killing of micro-organisms). An example of such an area is in the medical environment where surgical instruments, hospital consumables, such as bandages and surgical gloves, and also patient waste material require sterilisation.

### EXAMPLES

### Example 1- Heat inactivation of Bacillus subtilis spores

Spores of *Bacillus subtilis* 168 **[Bacillus genetic stock center:**
**http://www.bgsc.org/BGSCID:]** were generated by culture in synthetic MOPS medium (1.32 mM K₂HPO₄, 0.4 mM MgCl₂, 0.276 mM K₂SO₄, 0.01 mM FeSO₄, 1.36 mM CaCl₂, 80 mM MOPS (morpholinepropane sulfonic acid), 4 mM tricine, 3 nM (NH₄)₆Mo₇O₂₄, 0.4 µm H₃BO₃, 30 nM CoCl₂, 10 nM CuSO₄, 10 nM ZnCl₂, 0.1 mM MnCl₂, 20 mM glucose and 10 mM ammoniumchloride). The thus obtained spores were purified with 10 steps of washing with demineralised water at 4 °C. The purity of the spores was monitored with a phasecontrast microscope (>99% phase bright). Heat treatment of the spores was done according to Kooiman et al. ("The screw cap tube technique: A new and accurate technique for the determination of the wet heat resistance of bacterial spores". In: Spore Research, ed: Barker, A.N., Gould G.W. and Wolf, J., 1973, Academic Press, London, pp. 87-92). Survival was counted by plating serial dilutions of the samples in a peptone saline solution on TSA (tryptric soy agar, DIFCO, the Netherlands).

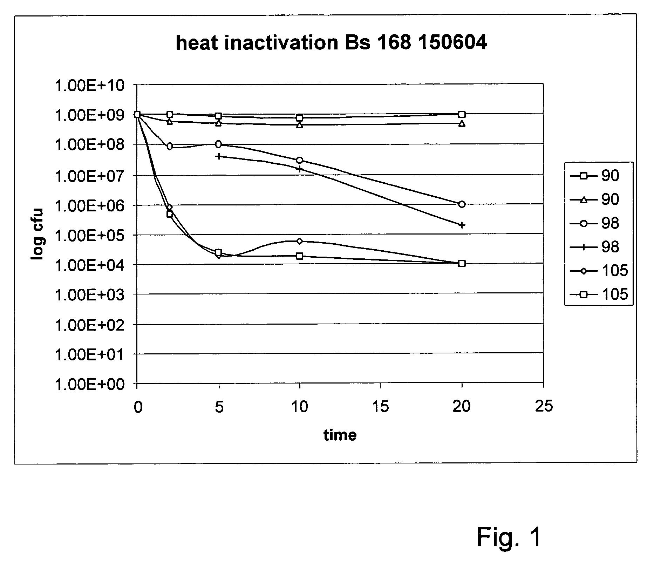

Results are shown in Fig. 1. It appears that spores of *B. subtilis* 168 were able to survive heat treatment of 90 °C, but that survival declines at temperatures of 98 °C and higher.

### Example 2 - Degradation of rRNA

From the heat treated spores of Example 1 the RNA was isolated with a BIO101-FASTRNA Pro Blue kit (QBiogene #6025-050) according to the manufacturer's instructions, with the modification that the spores were processed 3 times 40 seconds in the FastPrep apparatus (QBiogene #6001-220) on setting 6 with 2 minutes of cooling on ice in between the lysis steps. After precipitation the RNA was treated with the TURBO DNA-*free* kit (AMBION #1907) according to the manufacturer's instructions. The integrity of the RNA was analysed on a RNA 6000 Nano LabChip (Agilent Technologies #5065-4476) with a 2100 bioanalyzer (Agilent Technologies #G2940CA).

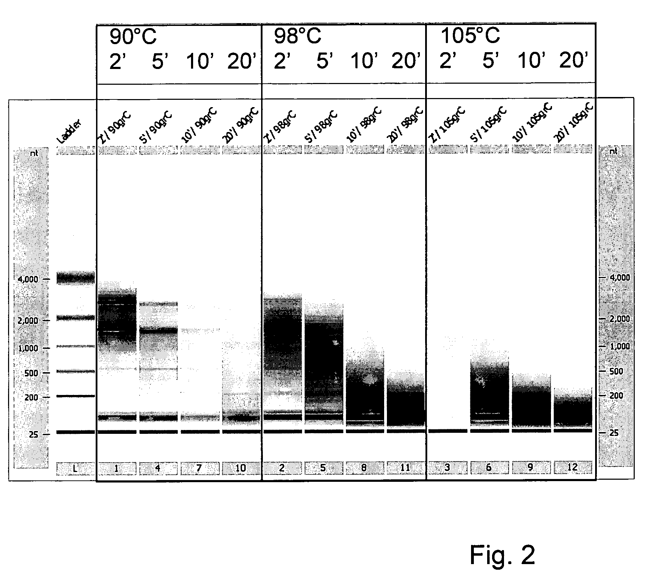

At relatively low temperature treatments two clear bands are visible on the Bioanalyzer pseudogel, which indicate the presence of 16S and 23S rRNA, in a about 1:1.8 ratio (Fig. 2). The intensity of the bands becomes weaker at higher temperatures and/or longer treatment times. This coincides with the appearance of RNA material of low molecular weight.

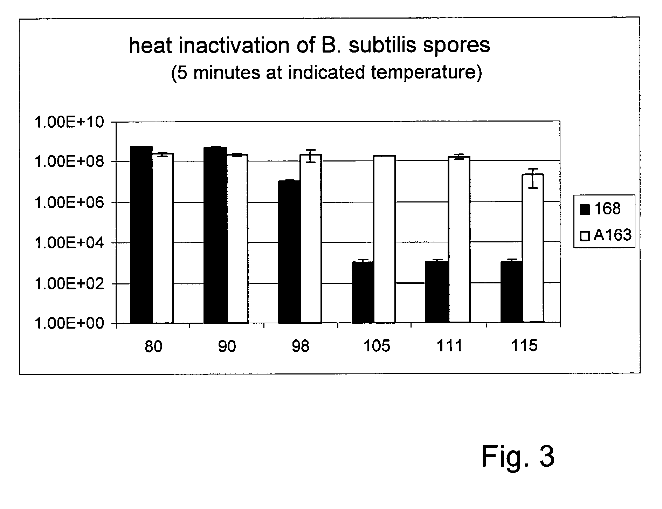

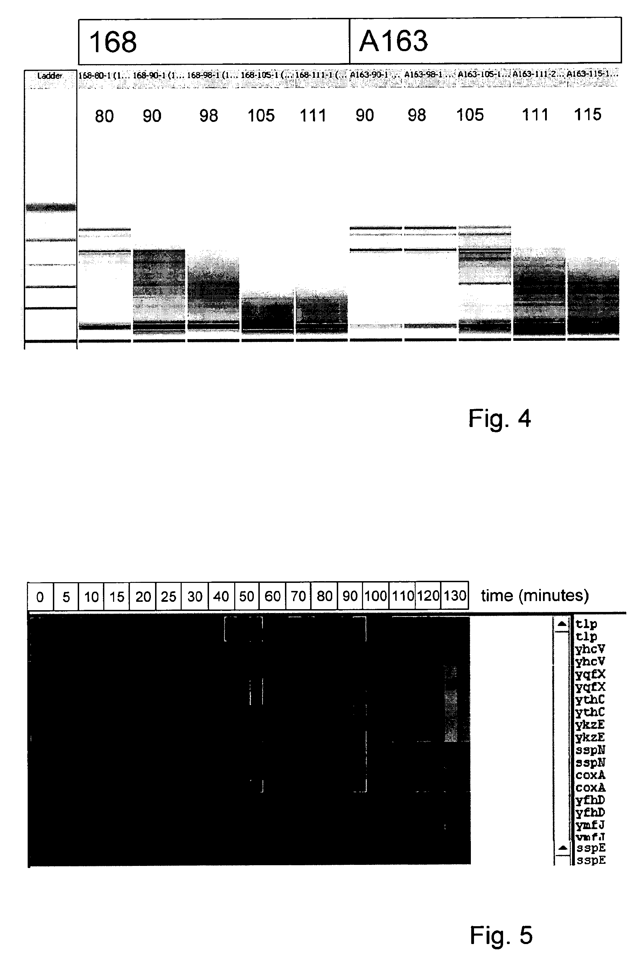

To investigate whether the degradation of RNA in the spores is dependent on the heat resistant properties of the spores, also the spores of a micro-organisms with a known higher heat resistance were tested. For this spores of the isolate *B. subtilis* A163 (Kort R, O'Brien AC, van Stokkum IH, Oomes SJ, Crielaard W, Hellingwerf KJ, Brul S. Assessment of heat resistance of bacterial spores from food product isolates by fluorescence monitoring of dipicolinic acid release. Appl Environ Microbiol. 2005 Jul;71(7):3556-64). were chosen. Spores were generated and heat treated in the same way as the *B*. *subtilis* 168 spores of Example 1. The results are shown in Figs. 3 and 4. While the viability of *B. subtilis* 168 spores decreases by 1 log after treatment at 98 °C for 5 minutes, there is no indication of loss of viability in *B. subtilis* A163 spores after this treatment. A comparable loss of viability in the latter spores only has been demonstrated at a temperature of 115 °C. The integrity of the rRNA from *B. subtilis* 168 remained intact at 80°C, but degradation was observed at temperatures of 90 °C and higher (in concordance with the previous experiment). In contrast, the integrity of the RNA isolated from *B*. *subtilis* A163 appeared intact after treatments up to 98 °C. With the 105 °C treatment degradation of the RNA was observed, predominantly of the 23S rRNA. Increasing degradation was observed at higher temperatures.

### Example 3 - Detection of mRNA in B. subtilis spores

Using microarray analysis germination of *B. subtilis* spores has been studied. Hereto, a *B. subtilis* oligonucleotide library (Sigma-Genosys #BACLIB96) was spotted onto Corning GAPSII slides (Corning, #40003) according to standard protocols. Cy-labeled cDNA of spore RNA was made by direct incorporation of cy-labeled dUTP according to standard protocols. Hybridisation and washing of the micro-arrays was performed on an automated slide processor (Agilent) and scanning of the micro-array slides was done in an automated slide scanner (Agilent) according to the manufacturer's instructions.

This showed (see Fig. 5) that a small group of mRNA molecules was present (about 20), of which the majority rapidly disappears during germination. Only *sspE* appeared to be present during the whole germination period. According to the fluorescence intensities of the hybridisation signals of the spore mRNAs the level of transcription was relatively high.

### Example 4 - Heat degradation of mRNA

### a. RT-PCR

To determine the stability of mRNA after heat treatment the spores were generated and treated as described in Example 1. RNA isolation was performed as described in Example 2. The isolated RNA was subjected to reverse transcription PCR (RT PCR) using Ready-to-Go RT-PCR Beads (Amersham Biosciences #27-9266-01) according to the manufacturer's instructions. Template concentration was between 10 and 200 ng. The primers used (determining 9 of the 20 transcription products) are indicated in Table 2.

**Table 2. RT-PCR Primer sequences**

| ***Gene*** | ***Primer forward*** | ***Primer reverse*** |
|---|---|---|
| TLP | TATCAGCAGCCTAATCCTG | CGTTTTGTCTCGCTGCAG |
| YHCV | GAGTTCAGTTAAAGATAC | ATACGAGTTCTGTTGACATC |
| YFHD | GGGCAGAAATCATATCC | CGCTCTGTTGTCGGCTG |
| YKZE | AACCGTCATAGCAGAGAC | TCAGGCTTGGTGACTTcc |
| SSPN | ATGGGAAACAACAAGAAAAAC | TCGCCTTTTGTCTGCATG |
| SSPE | GCTAACTCAAATAACTTCAGC | CAGCAGATTGGTTTTGCTG |
| COXA | GATACGCGCAATAACGGC | ATATGTTCCGTCAGTTGCC |
| YQFX | GAAGGTGGCAAACGATTAC | TGATGGACAAGGCTAAAGC |
| | | |

**Table 3. qPCR Primer and probe sequences. The dashes indicate superbases added for stability of the probe/primer, see http://www1.qiagen.com/Products/Pcr/QuantiTect/CustomAssays.aspx for details**

| Gene Name | Forward Primer | Reverse Primer | FAM-labeled Probe |
|---|---|---|---|
| *ykzE* | GCAGAGACA TGCAAAATCA TAA | CAT*T*GT*AAT*CC CCGAGTT | AGGTGCTGGAGGA AGAA |
| *coxA* | ATAGACAGG GAGACGGAA | TCGTCAGCAGTAA CACGGT | TAACCGAAACACCA CGA |
| *16s rRNA* | GGTCATTGG AAACTGGGA | CTACGCATTT*CAC CGCTA | GAAGAGGAGAGTG GAA |
| *23s rRNA* | CAGGTAACA CTGAATGGA | TTT*CGGAGAGAAC CAGCTAT | GATGAGGTGTGG*G TAG |

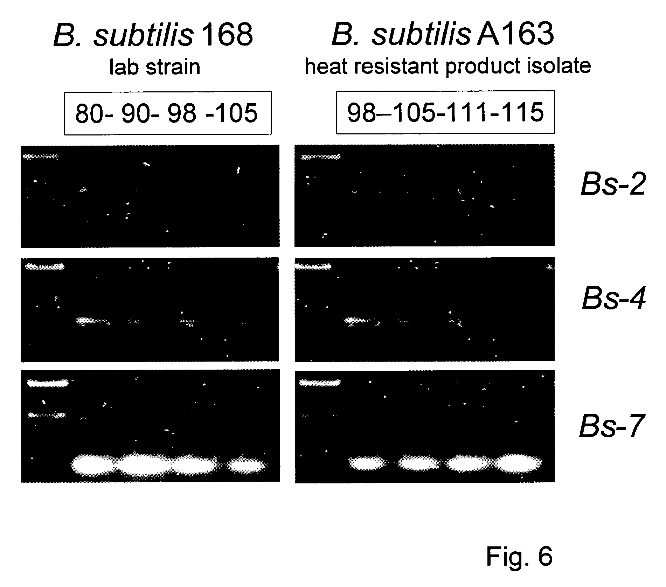

Products were analysed with agarose gel electrophoresis using staining with ethidium bromide (Fig. 6). In untreated *B. subtilis* 168 spores for all transcription products, except for *Tlp,* amplification products could be detected. In untreated *B. subtilis* A163 spores a clear amplification product was obtained with primers for *coxA, ykzE, yhcV, yfhD,* and *yqfX.* A weak signal was obtained with *sspE* primers and no product was formed using *Tlp* and *sspN* primers. Possibly these transcripts do not occur in spores of this *B*. *subtilis* strain, or the primersequences, which have been designed for *B. subtilis* 168 sequences, would not be suited for the A163 isolate.

In many cases in heat treated spores, a decrease in the amount of product was observed with increasing treatment temperatures (Fig. 6), which indicates degradation of mRNA. In spores of the A163 strain a similar decrease was observed, but starting at higher temperatures. For Bs-2 (the *coxA* transcript) no transcripts were detected in 168 spores at treatments temperatures of 98 °C and higher, while for A163 spores a decrease was only observed at temperatures of 115 °C. Bs-4 (*ykzE*) did not show a clear decrease in the amount of product, which may be an indication that this transcript is less sensitive to heat treatment. Alternatively, the effect could be caused by the quantitative character of the RT-PCR experiment. To investigate this further for a limited set of RNAs a quantitative PCR (qPCR) was performed.

### b. qPCR

*B. subtilis* 168 spores were heat treated at 98 °C, the survival was measured by plating and culturing, and the spore RNA was isolated as described above. Reverse transcription of the spore RNA was performed using a RETROscript kit (Ambion, #1710) according to the manufacturer's instructions. Quantitative PCR was performed using a QuantiTect Multiplex PCR kit (Qiagen, #204543) on a 7500 Real-Time PCR System (Applied Biosystems). Primers and probes (see Table 2) were designed using the QuantiProbe Design Software (http://customassays.qiagen.com/design/inputsequences.asp). Two primer-probe sets were used to study the effects on the rRNA (16S and 23S). Two primer-probe sets were directed against the *ykzE* and coxA spore transcripts. In this type of experiments, the number of amplification cycles (Ct) required to reach a certain threshold level, is used to calculate the original amount of template. A titration curve with genomic DNA was used to calibrate. A ten times reduction in template concentration appeared to increase the number of cycles necessary to reach the threshold by three. This calibration was used to interpret the differences in Ct values between the treated and untreated spores.

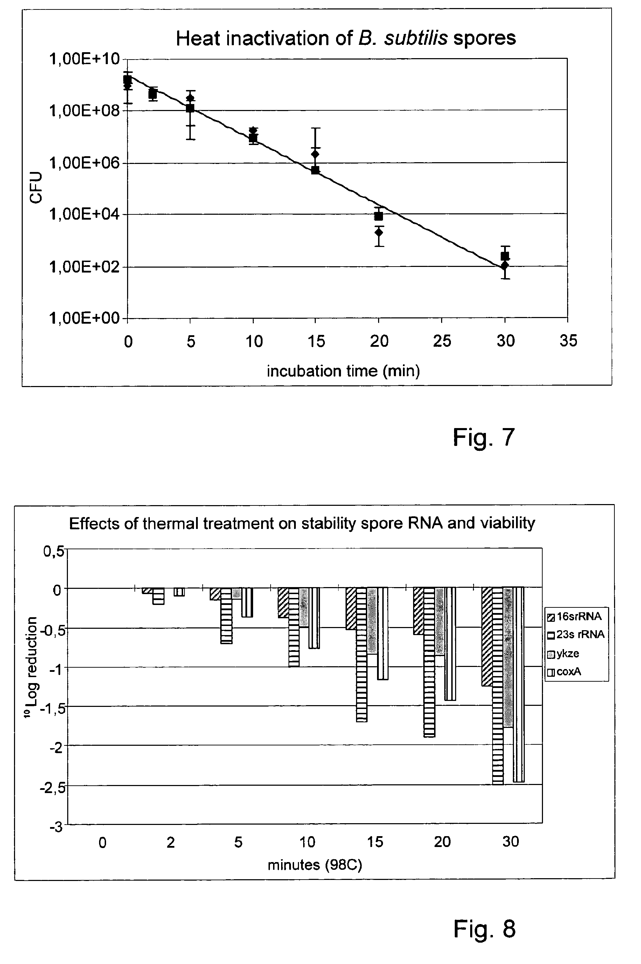

Fig. 7 shows the survival of heat treated spores according to the classical plating and culturing assay. Fig. 8 shows the results of the qPCR. It is clear that heat treatment results in a reduction of both the rRNAs and the mRNAs (which is in concordance with the earlier results on basis of gel electrophoresis). It also appears from this more sensitive qPCR that *ykzE,* which was deemed to be unresponsive in the RT-PCR assay, indeed degrades as a result of the heat treatment.

## Claims

1. Method to measure viability of heat, pressure or radiation treated bacterial spores comprising measuring the degree of degradation of RNA by said heat, pressure or radiation treatment in said spores.

2. Method according to claim 1,where in RNA is either ribosomal RNA (rRNA), messenger RNA (mRNA) or both

3. Method according to claim 1 or 2, wherein the treatment is heat treatment

4. Method to assay the effect of heat preservation methods by performing a method according to claim 1-3.

5. Method to assay bacterial contamination after heat or radiation preservation by performing a method according to claim 1-3

6. Heat preservation method comprising
a) maintaining a sample at a certain temperature for a certain time;
b) performing a method according to claim 1-3.

7. Use of RNA in microbial spores as a biomarker for viability.

8. Method for typing microbial spores comprising the steps of:
a) isolating the RNA form said spores;
b) optionally translate the RNA to DNA by reverse transcriptase and/or amplify the RNA or DNA; and
c) hybridising said RNA with type specific nucleotide probes or performing a sequence analysis of said RNA.

9. Method according to claim 8, wherein the RNA is mRNA


### Annex to the application documents - subsequently filed sequences listing

SEQUENCE LISTING

<110> Nederlandse Organisatie voor toegepast
natuurwetenschappelijk Onderzoek TNO

<120> Control of preservation by biomarkers

<130> P73012EP00

<140> EP 05077246.6
<141> 2005-10-04

<160> 28

<170> PatentIn version 3.3

<210> 1
<211> 19
<212> DNA
<213> Artificial

<220>
<223> Primer forward

<400> 1
tatcagcagc ctaatcctg

19

<210> 2
<211> 18
<212> DNA
<213> Artificial

<220>
<223> Primer reverse

<400> 2
cgttttgtct cgctgcag

18

<210> 3
<211> 18
<212> DNA
<213> Artificial

<220>
<223> Primer forward

<400> 3
gagttcagtt aaagatac

18

<210> 4
<211> 20
<212> DNA
<213> Artificial

<220>
<223> Primer reverse

<400> 4
atacgagttc tgttgacatc

20

<210> 5
<211> 17
<212> DNA
<213> Artificial

<220>
<223> Primer forward

<400> 5
gggcagaaat catatcc

17

<210> 6
<211> 17
<212> DNA
<213> Artificial

<220>
<223> Primer reverse

<400> 6
cgctctgttg tcggctg

17

<210> 7
<211> 18
<212> DNA
<213> Artificial

<220>
<223> Primer forward

<400> 7
aaccgtcata gcagagac

18

<210> 8
<211> 18
<212> DNA
<213> Artificial

<220>
<223> Primer reverse

<400> 8
tcaggcttgg tgacttcc

18

<210> 9
<211> 21
<212> DNA
<213> Artificial

<220>
<223> Primer forward

<400>  9
atgggaaaca acaagaaaaa c

21

<210>  10
<211>  18
<212>  DNA
<213>  Artificial

<220>
<223>  Primer reverse

<400>  10
tcgccttttg tctgcatg

18

<210>  11
<211>  21
<212>  DNA
<213>  Artificial

<220>
<223>  Primer forward

<400>  11
gctaactcaa ataacttcag c

21

<210>  12
<211>  19
<212>  DNA
<213>  Artificial

<220>
<223>  Primer reverse

<400>  12
cagcagattg gttttgctg

19

<210>  13
<211>  18
<212>  DNA
<213>  Artificial

<220>
<223>  Primer forward

<400>  13
gatacgcgca ataacggc

18

<210>  14
<211>  19
<212>  DNA
<213>  Artificial

<220>

<223>  Primer reverse

<400>  14
atatgttccg tcagttgcc

19


<210>  15
<211>  19
<212>  DNA
<213>  Artificial

<220>
<223>  Primer forward

<400>  15
gaaggtggca aacgattac

19


<210>  16
<211>  19
<212>  DNA
<213>  Artificial

<220>
<223>  Primer reverse

<400>  16
tgatggacaa ggctaaagc

19


<210>  17
<211>  22
<212>  DNA
<213>  Artificial

<220>
<223>  Forward primer

<400>  17
gcagagacat gcaaaatcat aa

22


<210>  18
<211>  18
<212>  DNA
<213>  Artificial

<220>
<223>  Reverse primer

<400>  18
cattgtaatc cccgagtt

18


<210>  19
<211>  17
<212>  DNA
<213>  Artificial

<220>
<223>  FAM-labeled probe

<400>  19
aggtgctgga ggaagaa

17


<210>  20
<211>  18
<212>  DNA
<213>  Artificial

<220>
<223>  Forward primer

<400>  20
atagacaggg agacggaa

18


<210>  21
<211>  19
<212>  DNA
<213>  Artificial

<220>
<223>  Reverse primer

<400>  21
tcgtcagcag taacacggt

19


<210>  22
<211>  17
<212>  DNA
<213>  Artificial

<220>
<223>  FAM-labeled probe

<400>  22
taaccgaaac accacga

17


<210>  23
<211>  18
<212>  DNA
<213>  Artificial

<220>
<223>  Forward primer

<400>  23
ggtcattgga aactggga

18


<210>  24
<211>  18

<212>  DNA
<213>  Artificial

<220>
<223>  Reverse primer

<400>  24
ctacgcattt caccgcta

18


<210>  25
<211>  16
<212>  DNA
<213>  Artificial

<220>
<223>  FAM-labeled probe

<400>  25
gaagaggaga gtggaa

16


<210>  26
<211>  18
<212>  DNA
<213>  Artificial

<220>
<223>  Forward primer

<400>  26
caggtaacac tgaatgga

18


<210>  27
<211>  20
<212>  DNA
<213>  Artificial

<220>
<223>  Reverse primer

<400>  27
tttcggagag aaccagctat

20


<210>  28
<211>  16
<212>  DNA
<213>  Artificial

<220>
<223>  FAM-labeled probe

<400>  28
gatgaggtgt gggtag

16